# EUROPEAN PATENT APPLICATION

(11) **EP 4 559 387 A1**
(43) Date of publication of application: **28.05.2025**
(21) Application number: 22952004.4
(22) Date of filing: 22.07.2022
(51) Int. Cl.: A61B 5/0538

(54) **SENSOR-ATTACHED GUIDE WIRE**

(71) Applicant: ASAHI INTECC CO., LTD., Seto-shi, Aichi, 489-0071 (JP)
(72) Inventor: NAMIMA, Satoshi, Seto-shi, Aichi 489-0071 (JP)
(74) Representative: Winter, Brandl - Partnerschaft mbB
(86) International application number: PCT/JP2022/028488
(87) International publication number: WO 2024/018620

(57) **Abstract**

A guide wire with a sensor includes: a core wire; a first coil body surrounding a distal end portion of the core wire; a distal tip that joints a distal end of the core wire and a distal end of the first coil body; and a tubular member provided with a sensor, in which the tubular member surrounds the core wire, a distal end portion of the tubular member is arranged inside a proximal end portion of the first coil body, and the distal end portion of the tubular member and the proximal end portion of the first coil body are jointed.

## Description

### TECHNICAL FIELD

The disclosed embodiment relates to a guide wire with a sensor.

### BACKGROUND ART

As a guide wire that guides a catheter or the like inserted to the inside of a living body lumen such as a blood vessel, there is a guide wire with a sensor in which the sensor is attached to a distal end portion. For example, Patent Literature 1 discloses a guide wire with a sensor including, in a distal end portion thereof, a housing with a sensor. In addition, Patent Literature 2 discloses a guide wire with a sensor including, in a distal end portion thereof, a sensor holder holding a sensor.

### CITATION LIST

### Patent Literature

Patent Literature 1: JP 2018-516623 W
Patent Literature 2: JP 2016-182213 A

### SUMMARY

### Technical Problem

When the center axis of the housing or the sensor holder disclosed in Patent Literatures 1 and 2 is shifted from the center axis of the guide wire with a sensor, torquability to the distal end portion is reduced, so that operability of the guide wire with a sensor is reduced. However, in Patent Literatures 1 and 2, there is no consideration of shifting between the center axis of the housing or the sensor holder and the center axis of the guide wire with a sensor. Therefore, development of a guide wire with a sensor in which torquability is improved in the distal end portion has been desired.

The disclosed embodiment has been made to solve at least a part of the above-mentioned problems, and are directed to provide a technology of improving torquability in a distal end portion of a guide wire with a sensor.

### Solution to Problem

The disclosed embodiment has been made to solve at least a part of the above-described problems, and can be realized as the following aspects.
(1) According to one aspect of the disclosed embodiment, a guide wire with a sensor is provided. This guide wire with a sensor includes: a core wire; a first coil body surrounding a distal end portion of the core wire; a distal tip that joints a distal end of the core wire and a distal end of the first coil body; and a tubular member provided with a sensor, in which the tubular member surrounds the core wire, a distal end portion of the tubular member is arranged inside a proximal end portion of the first coil body, and the distal end portion of the tubular member and the proximal end portion of the first coil body are jointed.
   According to this configuration, the distal end portion of the tubular member is arranged inside the proximal end portion of the first coil body, and the distal end portion of the tubular member and the proximal end portion of the first coil body are jointed to each other. Therefore, a center axis of the tubular member can be prevented from shifting from a center axis of the first coil body. Since the first coil body configures the contour of the distal end portion of the guide wire with a sensor, the center axis of the first coil body corresponds to the center axis in the distal end portion of the guide wire with a sensor. Therefore, in other words, the center axis of the tubular member can be prevented from shifting from the center axis of the guide wire with a sensor. Accordingly, the torquability in the distal end portion of the guide wire with a sensor is improved, and therefore, the operability of the guide wire with a sensor can be improved.
(2) In the guide wire with a sensor according to the mode described above, an outer diameter of the proximal end portion of the first coil body may be larger than an outer diameter of the tubular member including the sensor.
   With this configuration, it is possible to make the sensor provided in the tubular member or the tubular member hard to contact a living body tissue when the guide wire with a sensor is inserted to the inside of a living body lumen such as a blood vessel. As a result, the possibility that the sensor is damaged can be reduced and safety of the guide wire with a sensor with respect to a living body tissue can be improved.
(3) The guide wire with a sensor according to the mode described above may further include an inner coil body arranged between the first coil body and the core wire and surrounding the distal end portion of the core wire, in which a proximal end portion of the inner coil body may be arranged inside the distal end portion of the tubular member.
   With this configuration, since the proximal end portion of the inner coil body is arranged between the distal end portion of the tubular member and the core wire, the relative position of the tubular member with respect to the core wire can be prevented from shifting. Therefore, a center axis of the tubular member can be prevented from shifting from a center axis of the core wire. Accordingly, operability of the guide wire with a sensor can be further improved.
(4) The guide wire with a sensor according to the mode described above may further include a second coil body surrounding the core wire further to a proximal end side than the first coil body, in which a proximal end portion of the tubular member may be arranged inside a distal end portion of the second coil body, and the proximal end portion of the tubular member and the distal end portion of the second coil body may be jointed.
   With this configuration, a center axis of the tubular member can be prevented from shifting from a center axis of the second coil body. Since the second coil body configures a part of the contour of the guide wire with a sensor, the center axis of the second coil body corresponds to a part of the center axis of the guide wire with a sensor. Therefore, in other words, the center axis of the tubular member can be prevented from shifting from the center axis of the guide wire with a sensor. Accordingly, the torquability in a portion in the guide wire with a sensor the contour of which is configured by the second coil body is improved, and therefore, the operability of the guide wire with a sensor can be further improved.
(5) In the guide wire with a sensor according to the mode described above, an outer diameter of the distal end portion of the second coil body may be larger than an outer diameter of the tubular member including the sensor.

With this configuration, it is possible to make the sensor provided in the tubular member or the tubular member hard to contact a living body tissue when the guide wire with a sensor is inserted to the inside of a living body lumen such as a blood vessel. As a result, the possibility that the sensor is damaged can be reduced and safety of the guide wire with a sensor with respect to a living body tissue can be improved.

Note that the disclosed embodiments can be achieved in various aspects, such as a medical device including a guide wire with a sensor, a catheter including a guide wire with a sensor, and a manufacturing method of a guide wire with a sensor.

### BRIEF DESCRIPTION OF DRAWINGS

FIG. 1 is an explanatory view that illustrates an example of a configuration of a guide wire with a sensor of a first embodiment.
FIG. 2 is an enlarged view of a connector assembly.
FIGS. 3A to 3D are explanatory views of details of the connector assembly, a core wire, and a cable.
FIGS. 4A to 4C are cross-sectional views that show each cross section of a proximal end portion of the guide wire with a sensor.
FIG. 5 is an enlarged cross-sectional view of the sensor assembly.
FIG. 6 is an explanatory view for explaining details of a configuration of a tubular member.
FIG. 7 is an explanatory view for explaining details of a configuration of a sensor sheet.
FIG. 8 is an explanatory view for explaining a winding process of the sensor sheet with respect to the tubular member.
FIGS. 9A and 9B are explanatory views of details of the cable arranged in between a first section and a second section.
FIG. 10 is an explanatory view that shows a schematic configuration of a guide wire with a sensor of a second embodiment.
FIG. 11 is an enlarged view of a periphery of the sensor assembly.
FIG. 12 is an explanatory view that shows a schematic configuration of a guide wire with a sensor of a third embodiment.
FIG. 13 is a cross-sectional view that shows a cross section of a guide wire with a sensor of a fourth embodiment.
FIG. 14 is an explanatory view that shows a schematic configuration of a guide wire with a sensor of a fifth embodiment.

### DESCRIPTION OF EMBODIMENTS

### <First Embodiment>

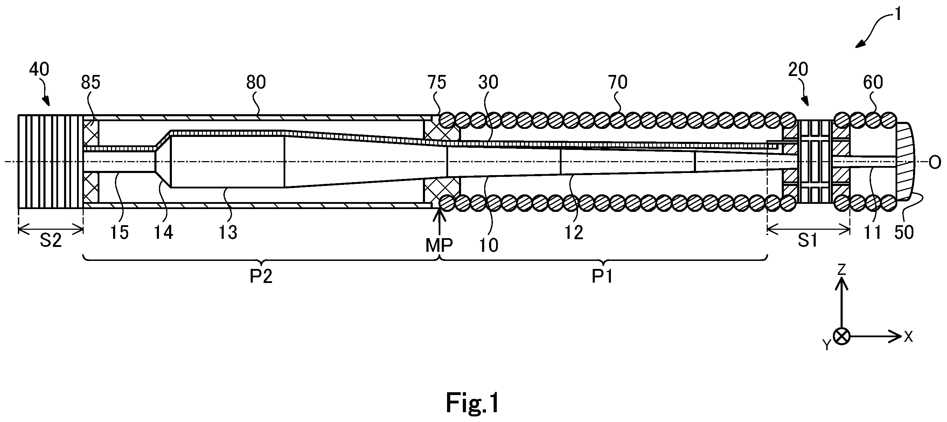

FIG. 1 is an explanatory view that illustrates an example of a cross-sectional configuration of a guide wire with a sensor 1 of a first embodiment. In FIG. 1, a sensor assembly 20 and a connector assembly 40 are shown by their appearance. The guide wire with a sensor 1 of the present embodiment is a device that is used by being inserted to a brain blood vessel, for example, and can measure electrical resistance of body fluid such as blood flowing through the blood vessel. The electrical resistance detected by the guide wire with a sensor 1 is used for, for example, determination of a type of a blood clot generated in the brain blood vessel.

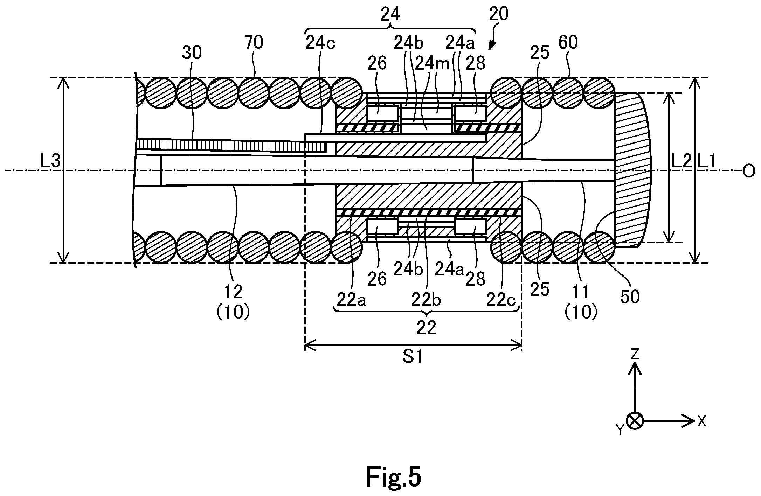

In FIG. 1, the axis passing through the center of the guide wire with a sensor 1 is represented by an axis O (dashed line). Hereinafter, description will be made assuming that the center axes passing centers of the core wire 10, the tubular member 22 (see FIG. 5), a first coil body 60, and a second coil body 70 at least described later among configuration members of the guide wire with a sensor 1 match the axis O. However, center axes passing centers of other members may be shifted from the axis O. Further, FIG. 1 illustrates XYZ axes that are orthogonal to each other. The X-axis corresponds to the axial direction of the guide wire with a sensor 1 (the insertion direction of the guide wire with a sensor 1), the Y-axis corresponds to the width direction of the guide wire with a sensor 1, and the Z-axis corresponds to the height direction of the guide wire with a sensor 1. The right side (+X-axis direction) in FIG. 1 is referred to as the "distal end side" of the guide wire with a sensor 1 and that of each configuration member, while the left side (-X-axis direction) in FIG. 1 is referred to as the "proximal end side" of the guide wire with a sensor 1 and that of each configuration member. Regarding the guide wire with a sensor 1 and each configuration member, an end portion located in the distal end side and the vicinity of the end portion are referred to as a "distal end portion" or simply "distal end", and an end portion located in the proximal end side and the vicinity of the end portion are referred to as a "proximal end portion" or simply "proximal end". In the guide wire with a sensor 1, the distal end side is a portion inserted to the inside of a living body, and the proximal end side is a portion operated by a surgeon such as a doctor. These points are also common to the figures following FIG. 1.

The guide wire with a sensor 1 includes a core wire 10, a sensor assembly 20, a cable 30, and a connector assembly 40. The core wire 10 is a long member extending along the X-axis direction. The core wire 10 has a distal end small diameter portion 11, a tapered portion 12, a large diameter portion 13, a reduced diameter portion 14, and a proximal end small diameter portion 15 in order from the distal end side to the proximal end side.

The distal end small diameter portion 11 is a portion that is provided in the most distal end side of the core wire 10, has a substantially cylindrical shape with a substantially constant outer diameter, and has the smallest outer diameter in the core wire 10. As used herein, "substantially constant" is synonymous with "generally constant", and means to be generally constant while allowing fluctuations due to manufacturing errors or the like. The tapered portion 12 is a portion that is provided in between the distal end small diameter portion 11 and the large diameter portion 13 and has an outer diameter gradually reduced from the proximal end side to the distal end side. The large diameter portion 13 is a portion that is provided in between the tapered portion 12 and the reduced diameter portion 14 and has a substantially columnar shape with an outer diameter larger than that of the distal end small diameter portion 11 or that of the proximal end small diameter portion 15. The reduced diameter portion 14 is a portion that is provided in between the large diameter portion 13 and the proximal end small diameter portion 15 and has an outer diameter gradually reduced from the distal end side to the proximal end side. The proximal end small diameter portion 15 is a portion that is provided adjacent to the reduced diameter portion 14 from the proximal end side, has a substantially columnar shape with a substantially constant outer diameter, and has an outer diameter smaller than that of the large diameter portion 13 in the core wire 10. The outer diameters, the lengths of the axis O direction, and the transverse sectional shapes of the distal end reduced diameter portion 11, the tapered portion 12, the large diameter portion 13, the small diameter portion 14, and the proximal end small diameter portion 15 can be determined as desired.

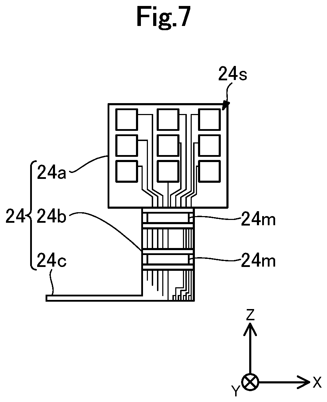

The sensor assembly 20 is arranged in the distal end portion of the core wire 10. The sensor assembly 20 is a structure body including a sensor array 24s (see FIG. 7) that measures electrical resistance of body fluid such as blood flowing through the inside of a blood vessel. The core wire 10 is inserted to the inside of the sensor assembly 20. Details of the sensor assembly 20 will be described with reference to FIGS. 5 to 8 described later.

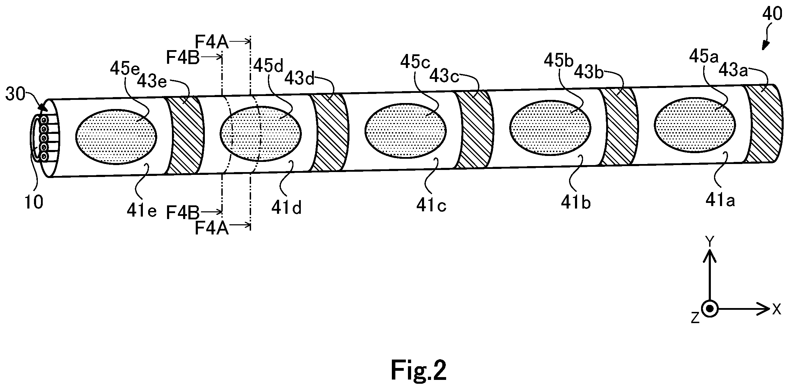

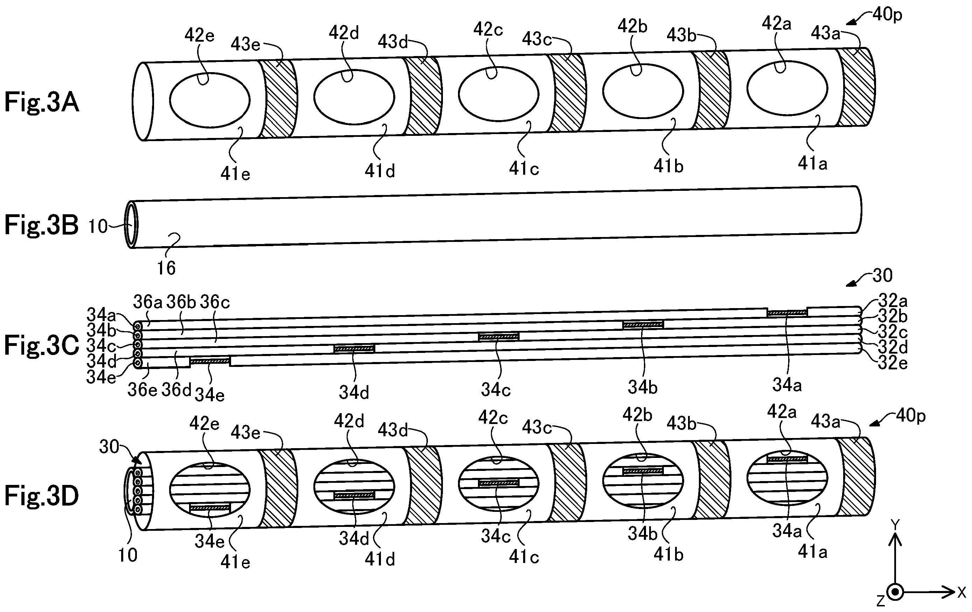

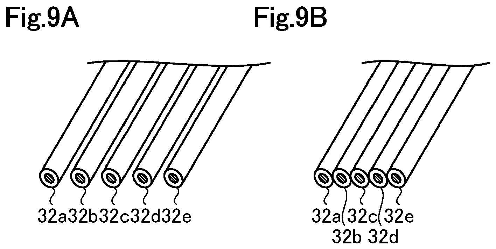

The cable 30 includes five lead wires 32a to e (see FIG. 3C or FIGS. 9A and B) extending to the proximal end portion of the core wire 10 along the core wire 10, and electrically connects a sensor wiring 24c (see FIG. 5) described later included in the sensor assembly 20 and hollow electrodes 41a to e (see FIG. 2) included in the connector assembly 40 described later. Details of the cable 30 will be described in FIGS. 3A to 3D and FIGS. 9A and 9B described later. "Electrically connect" refers to a connection in a state where a current can flow (conductive state).

The connector assembly 40 is arranged in the proximal end portion of the core wire 10. The connector assembly 40 is a structure body for connection with an external device that processes an output signal from the sensor array 24s (see FIG. 7) described later. The inside of the connector assembly 40 is arranged with a part of the proximal end small diameter portion 15 in the core wire 10 and the cable 30. Details of the connector assembly 40 will be described with reference to FIGS. 2 to 4 described later.

As shown in FIG. 1, the guide wire with a sensor 1 includes a distal tip 50, a first coil body 60, a second coil body 70, a middle joint member 75, a tube 80, and a proximal end side joint member 85.

The distal tip 50 joints the distal end of the core wire 10 and the distal end of the first coil body 60. The distal tip 50 can be formed of any adhesive such as a metal solder, e.g., silver brazing, gold brazing, zinc, Sn-Ag alloy, and Au-Sn alloy, and an epoxy adhesive.

The first coil body 60 and the second coil body 70 have flexibility and are substantially cylindrical shape having a substantially constant outer diameter from the proximal end side to the distal end side. The first coil body 60 is arranged so as to surround the distal end portion of the core wire 10. The second coil body 70 is arranged so as to surround the core wire 10 and the cable 30 further to the proximal end side from the first coil body 60. Specifically, the second coil body 70 is arranged so as to surround the core wire 10 and the cable 30 in the proximal end side from the sensor assembly 20. The first coil body 60 and the second coil body 70 are jointed to the sensor assembly 20. In the present embodiment, the first coil body 60 and the second coil body 70 are single-thread coils formed by winding one wire in a single thread. Note that the first coil body 60 and the second coil body 70 each may be a multi-thread coil formed by winding a plurality of wires in multiple threads, and may be a single-thread twisted wire coil formed by winding a twisted wire obtained by twisting a plurality of wires, in a single thread, or a multi-thread twisted wire coil formed by winding each twisted wire in multiple threads with the use of a plurality of twisted wires obtained by twisting a plurality of wires. The outer diameter and the inner diameter of the first coil body 60 and the second coil body 70 can be determined arbitrarily.

The tube 80 is a substantially cylindrical tube with a substantially constant outer diameter from the proximal end side to the distal end side. The tube 80 is arranged so as to surround the core wire 10 and the cable 30 further to the proximal end side than the second coil body 70. The tube 80 should be an antithrombotic property, flexibility, and biocompatibility, and can be formed of a resin material or a metal material. As the resin material, a polyimide resin, a polyamide resin, a polyolefin resin, a polyester resin, a polyurethane resin, a silicone resin, a fluororesin, and the like can be adopted, for example. Examples of the metal material that can be adopted herein include stainless steel such as SUS304, a nickel titanium alloy, and a cobalt chrome alloy. The outer surface of the tube 80 and the outer surfaces of the first coil body 60 and the second coil body 70 may be coated with a hydrophilic or hydrophobic resin.

The middle joint member 75 joints the core wire 10, the cable 30, the second coil body 70, and the tube 80. The proximal end side joint member 85 joints the core wire 10, the cable 30, the connector assembly 40, and the tube 80. As similar to the distal tip 50, any adhesive can be used for the middle joint member 75 and the proximal end side joint member 85. The first section S1, the second section S2, the middle position MP, the portion P1, and the portion P2 shown in FIG. 1 will be described later.

Next, details of the configuration of the connector assembly 40 will be described. FIG. 2 is an enlarged view of the connector assembly 40. As shown in FIG. 2, the connector assembly 40 arranged in the proximal end portion of the guide wire with a sensor 1 includes therein the core wire 10 and the cable 30. The connector assembly 40 includes hollow electrodes 41a to e, inter-ring joint members 43a to e, and blocking members 45a to e.

FIGS. 3A to 3D are explanatory views for explaining details of each of the connector assembly 40, and the core wire 10 and the cable 30 included inside of the connector assembly 40. FIG. 3A shows a structure body 40p obtained by excluding the blocking members 45a to e from the connector assembly 40. That is, the structure body 40p includes the hollow electrodes 41a to e and the inter-ring joint members 43a to e. Each of the hollow electrodes 41a to e is arranged in the proximal end portion of the core wire 10 and includes therein the core wire 10 and the cable 30 (see FIG. 2). The hollow electrodes 41a to e are arranged in the order of the hollow electrodes 41a, 41b, 41c, 41d, 41e from the distal end side to the proximal end side. In the present embodiment, the hollow electrodes 41a to e are annular electrodes. Each of the hollow electrodes 41a to e is formed with through holes 42a to e. As shown in FIG. 2, in the connector assembly 40, each of the through holes 42a to e is blocked by the blocking members 45a to e. The blocking members 45a to e are insulating member formed of a polyimide resin or the like.

The inter-ring joint members 43a to e are insulating joint members formed of a polyimide resin or the like. The inter-ring joint member 43a is arranged adjacently to the hollow electrode 41a from the distal end side, and joints the hollow electrode 41a and the tube 80 (see FIG. 1). Each of the inter-ring joint members 43b to e is arranged between the hollow electrodes 41a to e and joints each of the hollow electrodes 41a to e. Since the inter-ring joint members 43a to e formed of a resin are relatively flexible, the inter-ring joint members 43a to e are portions having low rigidity in the connector assembly 40. Therefore, a load generated when the connector assembly 40 is bent tends to be applied mainly to the inter-ring joint members 43a to e and hard to be applied to the hollow electrodes 41a to e having high rigidity.

FIG. 3B shows a portion in the core wire 10 arranged inside of the connector assembly 40. As shown in FIG. 3B, the portion in the core wire 10 arranged inside of the connector assembly 40 is covered with an insulating tube 16 formed of a polyimide rein or the like.

FIG. 3C shows a portion in the cable 30 arranged inside of the connector assembly 40. Five lead wires 32a to e constituting the cable 30 are arranged in the order to the lead wires 32a, 32b, 32c, 32d, and 32e from the +Y-axis direction side to the -Y-axis direction side (from the upper side in the drawing to the lower side in the drawing). The lead wires 32a to e respectively include core lines 34a to e and insulating tubes 36a to e. The core lines 34a to e are formed of a conductor. The insulating tubes 36a to e cover the core lines 34a to e, respectively. The insulating tubes 36a to e are formed of a polyimide resin or the like, as similar to the insulating tube 16 covering the core wire 10. As shown in FIG. 3C, portions of the insulating tubes 36a to e arranged inside of the connector assembly 40 is integrated with each other by outer peripheral surfaces of the portions being fused to each other. That is, in the portions in the cable 30 arranged inside of the connector assembly 40, the lead wires 32a to e are integrated with each other excluding a part of the portions. As shown in FIG. 3C, in a portion cable 30 arranged in the inside of the connector assembly 40, the entire periphery of parts of the insulating tubes 36a to e is peeled and the core lines 34a to e are exposed. The exposed portion corresponds to the part that is not integrated.

FIG. 3D is an explanatory view that illustrates a state where the structure body 40p includes therein the core wire 10 and the cable 30. The state shown in FIG. 3D corresponds to the state where only the blocking members 45a to e are excluded from the state shown in FIG. 2. As shown in FIG. 3D, the portion in the cable 30 in which the insulating tubes 36a to e are peeled and the core lines 34a to e are exposed is arranged inside of the through holes 42a to e. This state where the through holes 42a to e are blocked by the blocking members 45a to e is the state shown in FIG. 2.

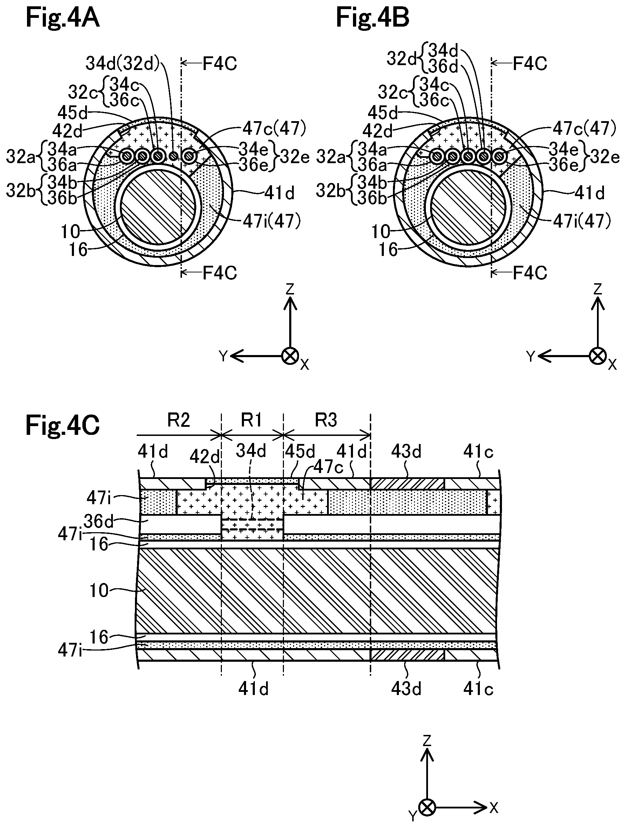

FIGS. 4A to 4C are cross-sectional views that show each cross section of the proximal end portion of the guide wire with a sensor 1. FIG. 4A shows a transverse section at line F4A-F4A in FIG. 2. The transverse section in FIG. 4A is a cross section in the portion in the cable 30 where the core line 34d is exposed. FIG. 4B shows a transverse section at line F4B-F4B in FIG. 2. The transverse section in FIG. 4B is a cross section in the portion in the cable 30 where the core line 34d is covered with the insulating tube 36d. FIG. 4C shows a vertical section at line F4C-F4C in FIGS. 4A and 4B. A first region R1 shown in FIG. 4C is a region inside of the hollow electrode 41d where the core line 34d exposed in the X-axis direction is arranged. That is, the cross-section of FIG. 4A is a cross section in the first region R1. In the vertical section shown in FIG. 4C, the core line 34d that cannot be viewed originally due to being hidden by the conductive connection member 47c described later is indicated by a dashed line. In the first region R1, in the lead wire 32d, the insulating tube 36d is peeled and the core line 34d is exposed, so that the lead wire 32d arranged in the first region R1 is configured by only the core line 34d. A second region R2 shown in FIG. 4C is a region different from the first region R1 inside of the hollow electrode 41d and is a region where the core line 34d covered with the insulating tube 36d is arranged in the X-axis direction. That is, the cross-section of FIG. 4B is a cross section in the second region R2. A third region R3 shown in FIG. 4C is a region inside of the hollow electrode 41d, in the side separated by the first region R1 from the second region R2, and is a region where the core line 34d covered with the insulating tube 36d is arranged in the X-axis direction as similar to the second region R2.

As shown in FIGS. 4A to 4C, a connection member 47 is arranged inside the hollow electrode 41d. The connection member 47 connects the core wire 10, the lead wires 32a to e, and the hollow electrode 41d. The connection member 47 includes a conductive connection member 47c and an insulating connection member 47i. The conductive connection member 47c is an adhesive containing metal powder and is mainly arranged in the periphery of a portion in which the core line 34d is exposed inside of the hollow electrode 41d. As shown in FIG. 4A, in the first region R1, the conductive connection member 47c covers at least a part of the lead wire 32d (configured by only the core line 34d in the first region R1) and electrically connects the lead wire 32d (core line 34d) and the hollow electrode 41d. In the present embodiment, the conductive connection member 47c covers a half or more of the outer periphery, specifically, the entire periphery of the outer periphery, of the transverse section (YZ cross section) of the lead wire 32d (core line 34d). The insulating connection member 47i is a non-conductive adhesive and is arranged so as to embed a portion inside of the hollow electrode 41d where the conductive connection member 47c is not arranged. The connection member 47 arranged as described above connects the core wire 10, the core line 34d, and the hollow electrode 41d in the first region R1. Specifically, as described in FIG. 3B, the core wire 10 arranged inside the connector assembly 40 is covered with the insulating tube 16, so that the connection member 47 connects the core wire 10 with the hollow electrode 41d and the core wire 10 with the core line 34d in an electrically insulated state in the first region R1. Since the connection member 47 is an adhesive and the first region R1 is filled with the connection member 47, the connection member 47 connects the core wire 10 with the hollow electrode 41d and the core wire 10 with the core line 34d in a state where relative positions are fixed.

As shown in FIG. 4A, in the first region R1, the lead wires 32c, 32e are separated from the lead wire 32d. Specifically, in the first region R1, as described above, the lead wire 32d is configured by only the core line 34d, and the lead wires 32c, 32e are separated from the core line 34d (lead wire 32d). In the first region R1, the connection member 47 connects the separated lead wire 32d (core line 34d) and the lead wires 32c, 32e, the core wire 10, and the hollow electrode 41d. On the other hand, as shown in FIG. 4B, in the second region R2, the lead wires 32c, 32e are integrated with the lead wire 32d. Specifically, in the second region R2, as described in FIG. 3C, the insulating tubes 36c to e have outer peripheral surfaces fused to each other, so that the lead wire 32d and the lead wires 32c, 32e are integrated with each other. In the second region R2, the connection member 47 connects the integrated lead wire 32d and the lead wires 32c, 32e, the core wire 10, and the hollow electrode 41d. In the connection state in the second region R2 described here is similar to the third region R3. Specifically, also in the third region R3, the lead wires 32c, 32e are integrated with the lead wire 32d, and the connection member 47 connects the integrated lead wire 32d and lead wires 32c, 32e, the core wire 10, and the hollow electrode 41d.

In the first region R1 inside of the hollow electrode 41d, the core line 34d is exposed and the lead wire 32d that is electrically connected with the hollow electrode 41d corresponds to a "main lead wire". The lead wires 32c, 32e that are separated from the lead wire 32d (main lead wire) in the first region R1 inside of the hollow electrode 41d and are integrated with the lead wire 32d (main lead wire) in the second region R2 inside of the hollow electrode 41d correspond to "auxiliary lead wires". Also inside of the hollow electrodes 41a to c, e, there are first to third regions as similar to the inside of the hollow electrode 41d and lead wires corresponding to the "main lead wires" and the "auxiliary lead wires". Specifically, the "main lead wire" in the hollow electrode 41a is the lead wire 32a, and the "auxiliary lead wire" is the lead wire 32b. The "main lead wire" in the hollow electrode 41b is the lead wire 32b, and the "auxiliary lead wire" is the lead wires 32a, c. The "main lead wire" in the hollow electrode 41c is the lead wire 32c, and the "auxiliary lead wire" is the lead wires 32b, d. The "main lead wire" in the hollow electrode 41e is the lead wire 32e, and the "auxiliary lead wire" is the lead wire 32b.

Next, details of the configuration of the sensor assembly 20 will be described. FIG. 5 is an enlarged cross-sectional view of the sensor assembly 20. The sensor assembly 20 includes a tubular member 22, a sensor sheet 24, and spacers 26, 28.

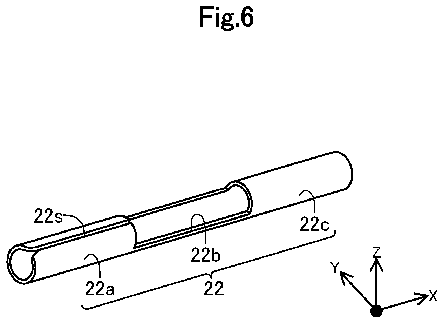

FIG. 6 is an explanatory view for explaining details of the configuration of the tubular member 22 being a part of the sensor assembly 20. The tubular member 22 is configured by the proximal end cylindrical portion 22a, a middle connection portion 22b, and a distal end cylindrical portion 22c. The proximal end cylindrical portion 22a is a cylindrical portion in the proximal end side in the tubular member 22. A side surface of the proximal end cylindrical portion 22a is formed with a slit 22s that communicates the inside and the outside of the proximal end cylindrical portion 22a. The middle connection portion 22b is a portion in the tubular member 22 that connects the proximal end cylindrical portion 22a and the distal end cylindrical portion 22c. The transverse section (YZ cross section) is a semicircular portion in which the inner surface of the middle connection portion 22b is exposed to the outside. The distal end cylindrical portion 22c is a cylindrical portion in the distal end side in the tubular member 22 and the transverse section (YZ cross section) has a substantially circular shape.

FIG. 7 is an explanatory view for explaining details of the configuration of the sensor sheet 24 being a part of the sensor assembly 20. The sensor sheet 24 is a sheet-shaped member wound around the tubular member 22. The sensor sheet 24 is configured by a large width portion 24a, a small width portion 24b, and a sensor wiring 24c. The large width portion 24a is a band-shaped portion having a width larger than the small width portion 24b. A sensor array 24s is arranged in the surface of the large width portion 24a. The sensor array 24s is configured by nine sensors arranged in a lattice shape. These sensors are sensors that measure electrical resistance of body fluid such as blood flowing through a blood vessel. The small width portion 24b is a band-shaped portion having a width smaller than the large width portion 24a and is connected with the large width portion 24a. Two microchips 24m connected with the sensor array 24s are arranged in the surface of the small width portion 24b. The sensor wiring 24c is a wiring that transmits a signal transmitted from the microchips 24m and is connected with the small width portion 24b in a portion in the small width portion 24b opposite to the side connected with the large width portion 24a.

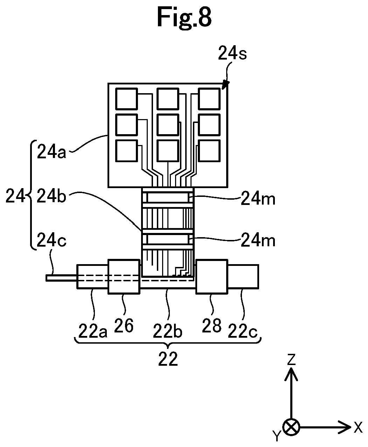

FIG. 8 is an explanatory view for explaining a winding process of the sensor sheet 24 with respect to the tubular member 22. The state shown in FIG. 8 is a state where the sensor wiring 24c is arranged inside the tubular member 22 via the slit 22s (not shown in FIG. 8, see FIG. 6) formed in the proximal end cylindrical portion 22a, thereby, the small width portion 24b projects from the middle connection portion 22b to the outside of the tubular member 22, and then, the annular spacers 26, 28 are attached to the tubular member 22. The annular spacers 26, 28 are attached to end portions in the middle connection portion 22b side in the proximal end cylindrical portion 22a and the distal end cylindrical portion 22c. In FIG. 8, a portion in the sensor sheet 24 arranged inside the tubular member 22 is indicated by a dashed line. From such a state, when the sensor sheet 24 is wound around the tubular member 22, first, the small width portion 24b in the sensor sheet 24 is wound around the middle connection portion 22b located between the spacers 26, 28. At this time, the two microchips 24m arranged in the surface of the small width portion 24b are wound around the middle connection portion 22b such that one is staked on the other (see FIG. 5). Thereafter, the large width portion 24a is wound around the small width portion 24b wound around the middle connection portion 22b and the outer peripheral surface of the spacers 26, 28, and thereby, the winding process of the sensor sheet 24 with respect to the tubular member 22 ends. After winding of the sensor sheet 24, nine sensors constituting the sensor array 24s are arranged in the outermost periphery of the tubular member 22. In the transverse section (YZ cross section) of the tubular member 22, three sensors are arranged along the circumferential direction at an interval of 120 degrees.

The description will be returned to FIG. 5. After the winding process described in FIG. 8, the tubular member 22 provided with the sensor sheet 24 in the circumference thereof is arranged so as to surround the core wire 10. The sensor assembly 20 further includes a distal end side joint member 25. The distal end side joint member 25 joints the core wire 10, the tubular member 22, the sensor sheet 24, the spacers 26, 28, the first coil body 60, and the second coil body 70. As similar to the middle joint member 75 and the proximal end side joint member 85, any adhesive can be used for the distal end side joint member 25.

In the present embodiment, as shown in FIG. 5, the distal end portion of the tubular member 22 (distal end cylindrical portion 22c) is arranged inside the proximal end portion of the first coil body 60, and the distal end portion of the tubular member 22 (distal end cylindrical portion 22c) and the proximal end portion of the first coil body 60 are jointed. As shown in FIG. 5, the outer diameter L1 of the proximal end portion of the first coil body 60 is larger than the outer diameter L2 of the tubular member 22 including the sensor sheet 24. The outer diameter L2 is the largest outer diameter of the tubular member 22 including the sensor sheet 24. For example, when the outer diameter at the position where the large width portion 24a is wound around the outer peripheral surface of the spacers 26, 28 corresponds to the outer diameter L2, the outer diameter L1 is larger than the outer diameter L2. On the other hand, as shown in FIG. 5, the proximal end portion of the tubular member 22 (proximal end cylindrical portion 22a) is arranged inside the distal end portion of the second coil body 70, and the proximal end portion of the tubular member 22 (proximal end cylindrical portion 22a) and the distal end portion of the second coil body 70 are jointed. As shown in FIG. 5, the outer diameter L3 of the distal end portion of the second coil body 70 is larger than the outer diameter L2 of the tubular member 22 including the sensor sheet 24.

Next, details of a portion in the cable 30 arranged between the sensor assembly 20 and the connector assembly 40 will be described. Before the cable 30 of the portion is described, the first section S1 and the second section S2 shown in FIG. 1 will be described. The first section S1 is a section located in the distal end portion of the core wire 10, in which the sensor assembly 20 is arranged in the X-axis direction. The proximal end of the first section S1 is a position of the proximal end of the sensor wiring 24c (see FIG. 5). The second section S2 is a section located in the proximal end portion of the core wire 10, in which the connector assembly 40 is arranged in the X-axis direction. The distal end of the second section S2 is at a position of the distal end of the inter-ring joint member 43a (see FIG. 2).

The cable 30 is electrically connected with the sensor wiring 24c in the first section S1 (see FIG. 5), and electrically connected with each of the hollow electrodes 41a to e in the second section S2. The electrical connection in the first section S1 is connection of a portion (not shown) in the distal end portion of the lead wires 32a to e constituting the cable 30, in which a part of the outer peripheral surface of each of the insulating tubes 36a to e is peeled and the core lines 34a to e are exposed, with each of five core line connection portions (not shown) provided in the sensor wiring 24c. As described in FIGS. 4A and 4C, the electrical connection in the second section S2 is connection via each of the lead wires 32a to e (core lines 34a to e) constituting the cable 30, each of the hollow electrodes 41a to e and the conductive connection member 47c.

FIGS. 9A and 9B are explanatory views for explaining details of a portion in the cable 30 arranged between the first section S1 and the second section S2. As shown in FIG. 1, any position between the proximal end of the first section S1 and the distal end of the second section S2 is set as a middle position MP. At this time, in the cable 30 arranged between the proximal end of the first section S1 and the middle position MP, as shown in FIG. 9A, all of the lead wires 32a to e in the portion therebetween (the portion between the proximal end of the first section S1 and the middle position MP) are not integrated with each other and are movable relatively to each other. In other words, in a portion in the cable 30 arranged between proximal end of the first section S1 and the middle position MP, since the outer peripheral surfaces of the insulating tubes 36a to e are separated and not fused to each other, the lead wires 32a to e are freely movable without following movement of each other. A length of a portion in the lead wires 32a to e arranged between the proximal end of the first section S1 and the middle position MP is longer than a length in the portion therebetween (the portion between the proximal end of the first section S1 and the middle position MP), so that the lead wires 32a to e are arranged in the state of being flexed to the portion therebetween. On the other hand, in the cable arranged between the middle position MP and the distal end of the second section S2, all of the lead wires 32a to e in the portion therebetween (the portion between the middle position MP and the distal end of the second section S2) are integrated with each other as shown in FIG. 9B. That is, in the portion in the cable 30 between the middle position MP and the distal end of the second section S2, since the outer peripheral surfaces of the insulating tubes 36a to e are fused to each other, the lead wires 32a to e are integrated with each other. As described above, in the portion between the proximal end of the first section S1 and the distal end of the second section S2, as shown in FIG. 1, the cable 30 is divided into a portion P1 where the lead wires 32a to e are not integrated with each other and a portion P2 where the lead wires 32a to e are integrated with each other with the middle position MP as a boundary. In the cable 30 electrically connected with the sensor wiring 24c in the distal end side from the proximal end of the first section S1, the lead wires 32a to e are integrated with each other. Also in the cable 30 in the proximal end side from the distal end of the second section S2, as described in FIG. 3C, the lead wires 32a to e are integrated with other excluding a part.

As shown in FIG. 1, in the present embodiment, the proximal end of the second coil body 70 in the guide wire with a sensor 1 corresponds to the middle position MP. Therefore, in the cable 30 arranged inside the second coil body 70, the lead wires 32a to e are not integrated with each other and are movable relatively. Since the middle position MP is the position included in the middle joint member 75, the cable 30 is jointed to the core wire 10 at the middle position MP. In between the sensor assembly 20 and the connector assembly 40, the cable 30 is jointed to the core wire 10 at the position where the middle joint member 75 and the proximal end side joint member 85 are arranged, and the cable 30 is not jointed to the core wire 10 at the position where the middle joint member 75 and the proximal end side joint member 85 are not arranged. In between the sensor assembly 20 and the connector assembly 40, the cable 30 is not wound around the core wire 10 and extends along the core wire 10.

In the present embodiment, in between the proximal end of the first section S1 and the middle position MP, the lengths of the lead wires 32a to e are equal to each other. Regarding the term "equal" herein, a manufacturing error or the like is allowed, and even when the lengths of the lead wires 32a to e are different from each other within a predetermined error range, they are described as being equal to each other.

As described above, in the present embodiment, the lead wires 32a to e are not integrated with each other in between the proximal end of the first section S1 and the middle position MP, and the lead wires 32a to e are integrated with each other in between the middle position MP and the distal end of the second section S2. Therefore, when one lead wire among the lead wires 32a to e is a "first lead wire", all of remaining four lead wires correspond to "second lead wires". That is, the "first lead wire" is a lead wire not integrated with the "second lead wire" in between the proximal end of the first section S1 and the middle position MP, and is a lead wire integrated with the "second lead wire" in between the middle position MP and the distal end of the second section S2.

As described above, according to the guide wire with a sensor 1 of the first embodiment, as shown in FIGS. 4A and 4C, the lead wire 32d and the hollow electrode 41d are electrically connected to each other in the first region R1. Therefore, when an external device that processes an output signal from the sensor array 24s and the hollow electrode 41d are connected to each other, the output signal from the sensor array 24s can be transmitted to the external device via the lead wire 32d connected to the sensor wiring 24c and the hollow electrode 41d. According to the guide wire with a sensor 1 of the first embodiment, as shown in FIGS. 4A and 4C, in the first region R1, the core wire 10 and the hollow electrode 41d are connected to each other and the core wire 10 and the lead wire 32d are connected to each other in an insulated state. Specifically, as shown in FIGS. 4A and 4C, while the connection member 47 covers the lead wire 32d separated from the lead wire 32c and the lead wire 32e, the connection member 47 connects the lead wire 32d with the hollow electrode 41d and also with the core wire 10. As a result, a change in a relative position of the hollow electrode 41d with respect to the core wire 10 can be prevented. Accordingly, it is possible to prevent a damage in the lead wire 32d due to the lead wire 32d being sandwiched between the core wire 10 and the hollow electrode 41d the relative positions of which have been changed. Since the core wire 10 is insulated from the hollow electrode 41d and the lead wire 32d, in the first region R1, it is also possible to prevent the output signal from the sensor array 24s from being transmitted to the core wire 10. The configurations and effects described herein are similar also in the first region inside of the hollow electrodes 41a to c, e.

For example, when the lead wire 32d is integrated with the lead wires 32c, 32e, a portion where the outer peripheral surface of the lead wire 32d and the outer peripheral surfaces of the lead wires 32c, 32e contact each other is not exposed to the outside. On the other hand, when the lead wire 32d is separated from the lead wires 32c, 32e, the entire outer peripheral surface of the lead wire 32d and the entire outer peripheral surfaces of the lead wires 32c, 32e are exposed to the outside. In the guide wire with a sensor 1 of the first embodiment, as shown in FIGS. 4A and 4B, the lead wire 32d is separated from the lead wires 32c, 32e in the first region R1, and the lead wire 32d is integrated with the lead wires 32c, 32e in the second region R2. Therefore, in the lead wire 32d and the lead wires 32c, 32e separated from each other in the first region R1, as compared to the lead wire 32d and the lead wires 32c, 32 integrated with each other, a connection area where the connection member 47 can be connected in each of the outer peripheral surface of the lead wire 32d and the outer peripheral surfaces of the lead wires 32c, 32e can be increased. Therefore, in the first region R1, the connection strength of the lead wire 32d and the lead wires 32c, 32e, and the core wire 10 and the hollow electrode 41d can be increased. On the other hand, in the second region R2, even when the lead wire 32d and the lead wires 32c, 32e and the core wire 10 and the hollow electrode 41d are disconnected due to peeling of the connection member 47 or the like, since the lead wire 32d and the lead wires 32c, 32e are integrated, the lead wire 32d and the lead wires 32c, 32e are prevented from being entangled. In addition, in the second region R2, the connection member 47 connects the lead wire 32d with the core wire 10 and the hollow electrode 41d in a state where the lead wire 32d is integrated with and lead wires 32c, 32e. Accordingly, separation of the lead wire 32d from the lead wires 32c and 32e in the first region R1 can be prevented from advancing to the second region R2. The configurations and effects described herein are similar also in the first region and the second region inside of the hollow electrodes 41a to c, e.

In the guide wire with a sensor 1 of the first embodiment, also in the third region R3, the lead wires 32c, 32e are integrated with the lead wire 32d. Therefore, as similar to the second region R2, also in the third region R3, when the lead wire 32d and the lead wires 32c, 32e and the core wire 10 and the hollow electrode 41d are disconnected, the lead wire 32d and the lead wires 32c, 32e are prevented from being entangled. In addition, also in the third region R3, the connection member 47 connects the lead wire 32d with the core wire 10 and the hollow electrode 41d in a state where the lead wire 32d is integrated with and lead wires 32c, 32e. Accordingly, separation of the lead wire 32d from the lead wires 32c and 32e in the first region R1 can be prevented from advancing to the third region R3. The configurations and effects described herein are similar also in the third region inside of the hollow electrodes 41a to c, e.

In the guide wire with a sensor 1 of the first embodiment, as shown in FIG. 4A, in the first region R1, the connection member 47 covers the entire outer periphery of the transverse section (YZ section) of the lead wire 32d. Therefore, it is possible to make the connection member 47 hard to be peeled from the lead wire 32d. Accordingly, it is possible to prevent disconnection of the lead wire 32d and the core wire 10 and disconnection of the lead wire 32d and the hollow electrode 41d due to peeling of the connection member 47 from the lead wire 32d. The configurations and effects described herein are similar also in the first region inside of the hollow electrodes 41a to c, e.

In the guide wire with a sensor 1 of the first embodiment, as shown in FIG. 5, the distal end portion of the tubular member 22 (distal end cylindrical portion 22c) is arranged inside the proximal end portion of the first coil body 60, and the distal end portion of the tubular member 22 (distal end cylindrical portion 22c) and the proximal end portion of the first coil body 60 are jointed. Therefore, a center axis of the tubular member 22 can be prevented from shifting from the center axis of the first coil body 60. Since the first coil body 60 forms the contour of the distal end portion of the guide wire with a sensor 1, the center axis of the first coil body 60 corresponds to the center axis in the distal end portion of the guide wire with a sensor 1. Therefore, in other words, the center axis of the tubular member 22 can be prevented from shifting from the center axis of the guide wire with a sensor 1. Accordingly, the torquability in the distal end portion of the guide wire with a sensor 1 is improved, and therefore, the operability of the guide wire with a sensor 1 can be improved.

In the guide wire with a sensor 1 of the first embodiment, as shown in FIG. 5, the outer diameter L1 of the proximal end portion of the first coil body 60 is larger than the outer diameter L2 of the tubular member 22 including the sensor sheet 24. Therefore, it is possible to make the sensor sheet 24 provided around the tubular member 22 or the tubular member 22 hard to contact a living body tissue when the guide wire with a sensor 1 is inserted to the inside of a living body lumen such as a blood vessel. As a result, the possibility that the sensor sheet 24 is damaged can be reduced and safety of the guide wire with a sensor 1 with respect to a living body tissue can be improved.

In the guide wire with a sensor 1 of the first embodiment, as shown in FIG. 5, the proximal end portion of the tubular member 22 (proximal end cylindrical portion 22a) is arranged inside the distal end portion of the second coil body 70, and the proximal end portion of the tubular member 22 (proximal end cylindrical portion 22a) and the proximal end portion of the second coil body 70 are jointed. Therefore, a center axis of the tubular member 22 can be prevented from shifting from the center axis of the second coil body 70. Since the second coil body 70 configures a part of the contour of the guide wire with a sensor 1, the center axis of the second coil body 70 corresponds to a part of the center axis of the guide wire with a sensor 1. Therefore, in other words, the center axis of the tubular member 22 can be prevented from shifting from the center axis of the guide wire with a sensor 1. Accordingly, the torquability in a portion in the guide wire with a sensor 1, the contour of which is configured by the second coil body 70 is improved, and therefore, the operability of the guide wire with a sensor 1 can be further improved.

In the guide wire with a sensor 1 of the first embodiment, as shown in FIG. 5, the outer diameter L3 of the distal end portion of the second coil body 70 is larger than the outer diameter L2 of the tubular member 22 including the sensor sheet 24. Therefore, it is possible to make the sensor sheet 24 provided around the tubular member 22 or the tubular member 22 hard to contact a living body tissue when the guide wire with a sensor 1 is inserted to the inside of a living body lumen such as a blood vessel. As a result, the possibility that the sensor sheet 24 is damaged can be reduced and safety of the guide wire with a sensor 1 with respect to a living body tissue can be improved.

In the guide wire with a sensor 1 of the first embodiment, in the cable 30 (see FIG. 1) arranged between the proximal end of the first section S1 and the middle position MP, as shown in FIG. 9A, the lead wires 32a to e are not integrated with each other and are movable relatively to each other. As a result, in between the proximal end of the first section S1 and the middle position MP, the lead wires 32a to e are freely movable without following movement of each other, so that flexibility of the distal end side of the guide wire with a sensor 1 can be secured. In the guide wire with a sensor 1 of the first embodiment, in the cable 30 (see FIG. 1) arranged between the middle position MP and the distal end of the second section S2, as shown in FIG. 9B, the lead wires 32a to e are integrated with each other. As a result, in between the middle position MP and the distal end of the second section S2, entanglement of the lead wires 32a to e can be prevented, so that operability of the proximal end side from the middle position MP in the guide wire with a sensor 1 can be secured. Therefore, according to the guide wire with a sensor 1 of the first embodiment, since both flexibility of the distal end side and operability of the proximal end side can be achieved, it is possible to provide the guide wire with a sensor 1 with improved handleability.

In between the proximal end of the first section S1 and the middle position MP, in the lead wires 32a to e not integrated with each other, it is possible to prevent generation of a winding habit in the core wire 10 due to pulling of the core wire 10 by the lead wires 32a to e caught by the second coil body 70. This is because, even when one of the lead wires 32a to e not integrated is caught by the second coil body 70, such a force of pulling that a winding habit is generated in the core wire 10 is hard to occur. In the lead wires 32a to e not integrated with each other, by arrangement in a state of being bent, disconnection due to rotation operation of the guide wire with a sensor 1 or insertion of the guide wire with a sensor 1 to a bent portion can be prevented.

In the guide wire with a sensor 1 of the first embodiment, in the cable 30 arranged inside the second coil body 70, the lead wires 32a to e are not integrated with each other and are movable relatively to each other. Therefore, since a flexible portion of the cable 30 in which the lead wires 32a to e are not integrated with each other is covered with the flexible second coil body 70, it is possible to provide the guide wire with a sensor 1 in which flexibility of the distal end side is secured.

In the guide wire with a sensor 1 of the first embodiment, the cable 30 is jointed to the core wire 10 at the middle position MP. Therefore, lead wires 32a to e integrated with each other in the proximal end side from the middle position MP are separated from each other by being pulled by the lead wires 32a to e not integrated with each other in the distal end side from the middle position MP. That is, with the middle position MP as a boundary, a portion where the lead wires 32a to e are not integrated with each other and a portion where the lead wires 32a to e are integrated can be maintained.

In the guide wire with a sensor 1 of the first embodiment, in between the proximal end of the first section S1 and the middle position MP, the lengths of the lead wires 32a to e are equal to each other. For example, in between the proximal end of the first section S1 and the middle position MP, when the length of the lead wire 32d is longer than the lengths of the lead wires 32a to c, e, the length of the lead wire 32d corresponds to a length obtained by adding a surplus length to the lengths of the lead wires 32a to c, e. As the length of the surplus length is longer, the possibility that the lead wire 32d is entangled with the lead wires 32a to c, e and the core wire 10 increases. Therefore, according to the guide wire with a sensor of the first embodiment, since the length of the lead wires 32a to e are equal to each other in between the proximal end of the first section S1 and the middle position MP, it is possible to reduce the possibility that lead wires 32a to e are entangled with each other or entangled with the core wire 10.

### <Second Embodiment>

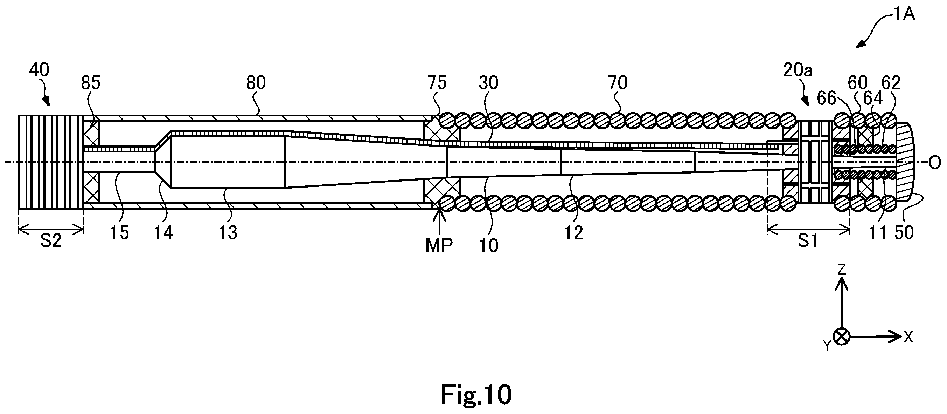

FIG. 10 is an explanatory view that shows a schematic configuration of a guide wire with a sensor 1A of a second embodiment. The guide wire with a sensor 1A of the second embodiment is different from the guide wire with a sensor 1 (FIG. 1) of the first embodiment in that the guide wire with a sensor 1A includes a sensor assembly 20a, an inner coil body 62, a first distal end joint member 64, and a second distal end joint member 66.

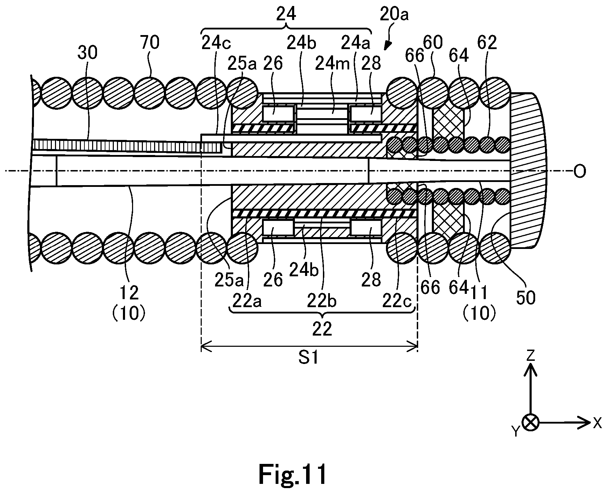

FIG. 11 is an enlarged view of a periphery of the sensor assembly 20a. The sensor assembly 20a is the same as the sensor assembly 20 of the first embodiment excluding a point that the sensor assembly 20a includes a distal end side joint member 25a different from the distal end side joint member 25 of the first embodiment. The distal end side joint member 25a joints the core wire 10, the tubular member 22, the sensor sheet 24, the spacers 26, 28, the first coil body 60, the second coil body 70, and the inner coil body 62.

The inner coil body 62 is arranged between the first coil body 60 and the core wire 10 and surrounds the distal end portion of the core wire 10. The proximal end portion of the inner coil body 62 is arranged inside the distal end portion of the tubular member 22 (distal end cylindrical portion 22c). The first distal end joint member 64 joints the first coil body 60 and the inner coil body 62. The second distal end joint member 66 joints the proximal end portion of the inner coil body 62 and the core wire 10. In the first distal end joint member 64 and the second distal end joint member 66, as similar to the distal end side joint member 25, the middle joint member 75 and the proximal end side joint member 85, any adhesive can be used for the distal end side joint member 25. For example, as the first distal end joint member 64 and the second distal end joint member 66, brazing material formed of silver, tin, copper, or the like or an adhesive may be used.

Such the above-described guide wire with a sensor 1A of the second embodiment can also exert similar effects as in the first embodiment. According to the guide wire with a sensor 1A of the second embodiment, since the proximal end portion of the inner coil body 62 is arranged between the distal end portion of the tubular member 22 (distal end cylindrical portion 22c) and the core wire 10, the relative position of the tubular member 22 with respect to the core wire 10 can be prevented from shifting. That is, a center axis of the tubular member 22 can be prevented from shifting from the center axis of the core wire 10. Accordingly, torquability of the guide wire with a sensor 1A is improved and operability can be further improved.

### <Third Embodiment>

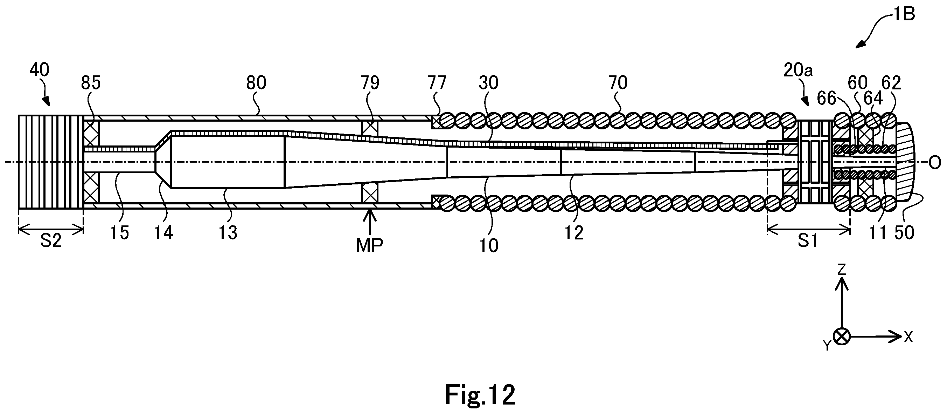

FIG. 12 is an explanatory view that shows a schematic configuration of a guide wire with a sensor 1B of a third embodiment. The guide wire with a sensor 1B of the third embodiment is different from the guide wire with a sensor 1A (FIG. 10) of the second embodiment in that the position of the middle position MP is different and the guide wire with a sensor 1B includes a first middle joint member 77 and a second middle joint member 79 instead of the middle joint member 75 of the first and second embodiments.

As described in the first embodiment, the middle position MP is a boundary between a portion where the lead wires 32a to e are not integrated with each other and a portion where the lead wires 32a to e are integrated can be maintained. In the guide wires with a sensor 1, 1A of the first and second embodiments, the middle position MP is a position in the proximal end of the second coil body 70. However, in the guide wire with a sensor 1B of the third embodiment, the middle position MP is provided further to the proximal end side than the proximal end of the second coil body 70. The first middle joint member 77 joints the second coil body 70 and the tube 80. The second middle joint member 79 is arranged so as to include the middle position MP, and joints the core wire 10, the cable 30, and the tube 80. That is, also in the guide wire with a sensor 1B of the third embodiment, the cable 30 is jointed to the core wire 10 at the middle position MP.

Such the above-described guide wire with a sensor 1B of the third embodiment can also exert similar effects as in the first embodiment. According to the guide wire with a sensor 1B of the third embodiment, it is possible to provide the guide wire with a sensor 1B in which flexibility is secured further to the proximal end side than the proximal end of the second coil body 70.

### <Fourth Embodiment>

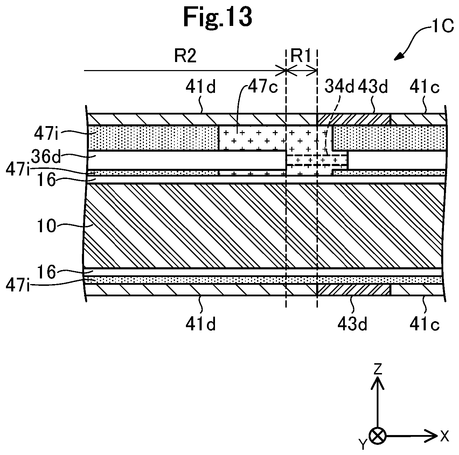

FIG. 13 is a cross-sectional view that shows a cross section of a guide wire with a sensor 1C of a fourth embodiment. The cross section of FIG. 13 is a cross section of the guide wire with a sensor 1C of the fourth embodiment corresponding to the cross section of the guide wire with a sensor 1 of the first embodiment shown in FIG. 4C. The guide wire with a sensor 1C of the fourth embodiment is the same as the guide wire with a sensor 1 of the first embodiment mainly excluding points that the arrangement of the first region inside of the hollow electrodes 41a to e (a region where the core lines 34a to e exposed are arranged in the X-axis direction) is different and that the through holes 42a to e are not formed in the hollow electrodes 41a to e.

In the guide wire with a sensor 1 of the first embodiment, for example, when the hollow electrode 41d is used for description, as shown in FIG. 4C, the entire core line 34d exposed in the X-axis direction is arranged inside the hollow electrode 41d. However, in the guide wire with a sensor 1C of the fourth embodiment, as shown in FIG. 13, a part of the core line 34d exposed in the X-axis direction is arranged inside the hollow electrode 41d and the other part is arranged inside the inter-ring joint member 43d. A portion in the core line 34d exposed in the X-axis direction arranged inside the hollow electrode 41d corresponds to the first region R1. With such arrangement of the first region R1, in the guide wire with a sensor 1C of the fourth embodiment, although there is the second region R2, there is no third region R3. Although the arrangement of the first region R1 is different, as similar to the first embodiment, the conductive connection member 47c is arranged in the periphery of the core line 34d exposed in the first region R1. The conductive connection member 47c is arranged also in the periphery of the core line 34d arranged inside the inter-ring joint member 43d.

Such the above-described guide wire with a sensor 1C of the fourth embodiment can also exert similar effects as in the first embodiment. According to the guide wire with a sensor 1C of the fourth embodiment, since the through holes 42a to e are not formed in the hollow electrodes 41a to e, it is possible to provide the guide wire with a sensor 1C including the connector assembly 40 having high rigidity.

### <Fifth Embodiment>

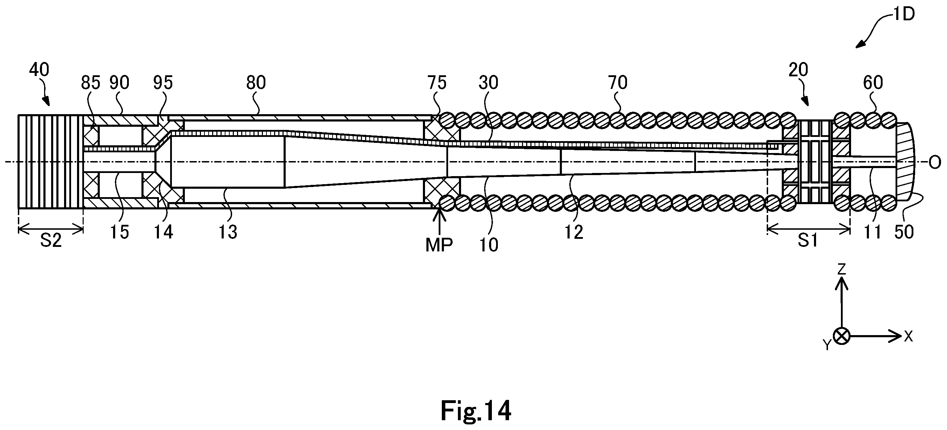

FIG. 14 is an explanatory view that shows a schematic configuration of a guide wire with a sensor 1D of a fifth embodiment. The guide wire with a sensor 1D of the fifth embodiment is different from the guide wire with a sensor 1 (FIG. 1) of the first embodiment in that the guide wire with a sensor 1D further includes a proximal end tube 90 and a tube joint member 95.

In the guide wire with a sensor 1D of the fifth embodiment, as compared to the guide wire with a sensor 1A (FIG. 1) of the first embodiment, the length of the tube 80 in the X-axis direction is short. The proximal end tube 90 is arranged in between the tube 80 being short as described above and the connector assembly 40. As similar to the tube 80, the proximal end tube 90 is a substantially cylindrical tube with a substantially constant outer diameter from the proximal end side to the distal end side. The proximal end tube 90 is formed of a material having higher strength than that of the tube 80. The tube joint member 95 joints the tube 80 and the proximal end tube 90.

Such the above-described guide wire with a sensor 1D of the fifth embodiment can also exert similar effects as in the first embodiment. According to the guide wire with a sensor 1D of the fifth embodiment, since the proximal end tube 90 having higher strength than that of the tube 80 is arranged in the proximal end side, it is possible to provide the guide wire with a sensor 1D with improved operability of the proximal end side.

### <Modification examples of the embodiments>

The disclosed embodiments are not limited to those above, and can be implemented in various aspects without departing from the gist thereof. For example, the following modification examples are also possible.

### [Modification example 1]

In the first to fifth embodiments, the configurations of the guide wires with a sensor 1, 1A to 1D are illustrated. However, various modifications can be made to the configurations of the guide wires with a sensor. For example, the guide wires with a sensor 1, 1A to D each include a sensor that measures electrical resistance of body fluid such as blood flowing through a blood vessel, but the disclosed embodiment is not limited thereto. For example, the guide wire with a sensor may include a sensor that measures a blood pressure (internal pressure of a blood vessel). The blood pressure measured by such a sensor is used for deriving a fractional flow reserve (FFR). The FFR is a pressure in the rear of stenosis with respect to a pressure in the front of stenosis and can be used as an indicator of severity of physiological stenosis.

### [Modification example 2]

In the embodiments described above, as shown in FIG. 3C, in a portion in the cable 30 arranged inside of the connector assembly 40, the entire periphery of each part of the insulating tubes 36a to e is peeled and the core lines 34a to e are exposed and the exposed portion is made, but the disclosed embodiment is not limited thereto. For example, in each part of the insulating tubes 36a to e, a part of the entire periphery circumference is peeled (that is, in a state where the insulating tubes 36a to e are remained in a portion other than the peeled portion in the entire circumference), the core lines 34a to e may be the exposed portion.

### [Modification example 3]

In the above-described embodiments, as shown in FIGS. 4A to 4C, inside of the hollow electrode 41d, the connection member 47 is arranged with no gap (the inside is filled with the connection member 47), but the disclosed embodiment is not limited thereto. That is, in the first region R1, the connection member 47 may be arranged as desired inside of the hollow electrode 41d as long as the connection member 47 electrically connects the lead wire 32d (core line 34d) and the hollow electrode 41d, and connects the core wire 10 and the hollow electrode 41d and the core wire 10 and the core line 34d in an electrically insulated state. For example, inside of the hollow electrode 41d, the connection member 47 may be arranged so as to have a gap, and the connection member 47 may be arranged so as not to be connected with each other in the first to third regions R1 to R3 and so as to be independent in each region. When the portion in the core wire 10 arranged inside the connector assembly 40 is covered with the insulating tube 16, the connection member 47 can be configured by only the conductive connection member 47c without including the insulating connection member 47i. On the other hand, when the portion in the core wire 10 arranged inside the connector assembly 40 is not covered with the insulating tube 16, inside of the hollow electrode 41d, the connection member 47 can be arranged such that, while the periphery of the core wire 10 is covered with the insulating connection member 47i, the other portion is filled with the conductive connection member 47c. In the first embodiment, the conductive connection member 47c is arranged so as to cover the entire outer periphery of the transverse section (YZ cross section) of the core line 34d, but the disclosed embodiment is not limited thereto. As long as the conductive connection member 47c is arranged so as to connect at least a part of the outer periphery of the transverse section (YZ cross section) of the core line 34d and the hollow electrode 41d, the conductive connection member 47c may be arranged as desired.

### [Modification example 4]

In the above-described embodiments, as shown in FIG. 2, each of the hollow electrodes 41a to e is formed with the through holes 42a to e. However, the number of through holes is not limited to one and each of the hollow electrodes 41a to e may be formed with two or more through holes. When two or more through holes are formed, portions where the insulating tubes 36a to e are peeled and the core lines 34a to e are exposed may be formed by the number equal to the number of through holes and arranged inside of the through holes.

### [Modification example 5]

In the above-described embodiments, the sensor sheet 24 being a sheet-shaped member is wound and provided around the tubular member 22. However, the disclosed embodiment is not limited thereto. The sensor may not be a sheet-shaped member and may be attached to the tubular member 22 in any method as long as the sensor is provided in the tubular member 22. For example, the sensor may be attached directly to the outer peripheral surface or the inner peripheral surface of the tubular member 22, or may be attached indirectly via another member.

### [Modification example 6]

In the above-described embodiments, as shown in FIGS. 5 and 11, the cable 30 and the sensor wiring 24c are connected outside of the tubular member 22 (further to the proximal end side than the proximal end of the tubular member 22). However, they may be connected inside of the tubular member 22.

### [Modification example 7]

In the above-described embodiment, the second coil body 70 is arranged as the tubular member surrounding the core wire 10 and the cable 30 further to the proximal end side than the first coil body 60, but the disclosed embodiment is not limited thereto. For example, as long as the second coil body 70 has flexibility as the tubular member surrounding the core wire 10 and the cable 30 further to the proximal end side than the first coil body 60, the second coil body 70 may be a substantially cylindrical tube or may be a tube formed with a spiral slit in the outer peripheral surface thereof.

### [Modification example 8]

In the above-described embodiments, in the cable 30 arranged between the proximal end of the first section S1 and the middle position MP, all of the lead wires 32a to e in the portion therebetween (the portion between the proximal end of the first section S1 and the middle position MP) are not integrated with each other. However, only a part of the lead wires 32a to e in the portion therebetween may not be integrated with each other. For example, in the lead wires 32a to e in the portion therebetween, only the portion between the position further to the proximal end side than the proximal end of the first section S1 and the middle position MP may not be integrated with each other, or not-integrated portions may be dispersed in the lead wires 32a to e in the portion therebetween.

### [Modification example 9]

In the above-described embodiments, the lead wires 32a to e are not integrated with each other in between the proximal end of the first section S1 and the middle position MP (here, a distal end section), and the lead wires 32a to e are integrated with each other in between the middle position MP and distal end of the second section S2, but the disclosed embodiment is not limited thereto. For example, the lead wires 32a to e may not be integrated with each other in the distal end section, and, among the lead wires 32a to e, the combination of the lead wires 32a and b and the combination of the lead wires 32c to e may be integrated with each other separately in the proximal end section. The lead wires 32a and b may not be integrated with each other in the distal end section but be integrated with each other in the proximal end section, and the lead wires 32c to e may be integrated with each other in both the distal end section and the proximal end section. That is, as long as the number of integrated lead wires in the distal end section is smaller than that of the proximal section, the number of lead wires that are integrated with the other lead wire in the proximal end section and are not integrated with the other lead wire in the distal end section may be set as desired.

### [Modification example 10]

In the above-described embodiments, as described in FIG. 3C and FIGS. 9A and 9B, integration of the lead wires is achieved by the outer peripheral surfaces of the insulating tubes being fused to each other, but the disclosed embodiment is not limited thereto. For example, the integration of the lead wires may be achieved by bundling two or more lead wires with an extensible annular member or by arranging a lead wire inside of the cylindrical member enclosing two or more lead wires.

### [Modification example 11]

In the first embodiment, the cable 30 (the portion P2 where the lead wires 32a to e are integrated) and the core wire 10 are not jointed to each other in between the middle joint member 75 and the proximal end side joint member 85, but the disclosed embodiment is not limited thereto. For example, the cable 30 (the portion P2 where the lead wires 32a to e are integrated) and the core wire 10 may be jointed in between the middle joint member 75 and the proximal end side joint member 85 by an adhesive or the like.

### [Modification example 12]

In the first embodiment, in between the sensor assembly 20 and the connector assembly 40, the cable 30 is not wound around the core wire 10 and extends along the core wire 10, but the disclosed embodiment is not limited thereto. For example, the portion P2 where at least the lead wires 32a to e in the cable 30 are integrated with each other (the portion between the middle joint member 75 and the proximal end side joint member 85) may be wound around the core wire 10.

### [Modification example 13]

In the first to third embodiments, instead of the tube 80, the proximal end tube 90 may be arranged. That is, the core wire 10 and the cable 30 in between the middle joint member 75 and the proximal end side joint member 85 may be surrounded by the proximal end tube 90.

### [Modification example 14]

The configurations of the guide wires with a sensor 1, 1A to 1D of the first to fifth embodiments, and each configuration of the above modification examples 1 to 8 may be combined appropriately. For example, in the guide wire with a sensor 1 of the first embodiment, as described in the third embodiment, the middle position MP may be provided further to the proximal end side than the proximal end of the second coil body 70. In the guide wires with a sensor 1A, 1B of the second and third embodiments, as described in the fourth embodiment, the through holes 42a to e may not be formed in the hollow electrodes 41a to e, and, as described in the fifth embodiment, the proximal end tube 90 and the tube joint member 95 may be provided.

The present aspects are described above on the basis of the embodiments and the modification examples. However, the embodiments of the aforementioned aspects are provided to facilitate the understanding of the present aspects, and do not limit the present aspects. The present aspects may be altered or improved without departing from the spirit thereof and claims, and the present aspects include their equivalents. Further, the technical features thereof, if not indicated as essential in the present specification, may be appropriately deleted.

### [Reference Signs List]

1, 1A to D Guide wire
10 Core wire
11 Distal end small diameter portion
12 Tapered portion
13 Large diameter portion
14 Reduced diameter portion
15 Proximal end small diameter portion
16 Insulating tube
20, 20a Sensor assembly
22 Tubular member
22a Proximal end cylindrical portion
22b Middle connection portion
22c Distal end cylindrical portion
22s Slit
24 Sensor sheet
24a Large width portion
24b Small width portion
24c Sensor wiring
24m Microchip
24s Sensor array
25, 25a Distal end side joint member
26 Spacer
30 Cable
32a to e Lead wires
34a to e Core lines
36a to e Insulating tube
40 Connector assembly
40p Structure body
41a to e Hollow electrode
42a to e Through hole
43a to e inter-ring joint member
45a to e Blocking member
47 Connection member
47c Conductive connection member
47i Insulating connection member
50 Distal tip
60 First coil body
62 Inner coil body
64 First distal end joint member
66 Second distal end joint member
70 Second coil body
75 Middle joint member
77 First middle joint member
79 Second middle joint member
80 Tube
85 Proximal end side joint member
90 Proximal end tube
95 Tube joint member

## Claims

1. A guide wire with a sensor comprising:
a core wire;
a first coil body surrounding a distal end portion of the core wire;
a distal tip that joints a distal end of the core wire and a distal end of the first coil body; and
a tubular member provided with a sensor,
wherein the tubular member surrounds the core wire, a distal end portion of the tubular member is arranged inside a proximal end portion of the first coil body,
and the distal end portion of the tubular member and the proximal end portion of the first coil body are jointed to each other.

2. The guide wire with a sensor according to claim 1,
wherein an outer diameter of the proximal end portion of the first coil body is larger than an outer diameter of the tubular member including the sensor.

3. The guide wire with a sensor according to claim 1 or 2, further comprising:
an inner coil body arranged between the first coil body and the core wire and surrounding the distal end portion of the core wire,
wherein a proximal end portion of the inner coil body is arranged inside the distal end portion of the tubular member.

4. The guide wire with a sensor according to any one of claims 1 to 3, further comprising
a second coil body surrounding the core wire in a proximal end side from the first coil body,
wherein a proximal end portion of the tubular member is arranged inside a distal end portion of the second coil body, and
the proximal end portion of the tubular member and the distal end portion of the second coil body are jointed.

5. The guide wire with a sensor ccording to claim 4,
wherein an outer diameter of the distal end portion of the second coil body is larger than the outer diameter of the tubular member including the sensor.
